# EUROPÄISCHE PATENTSCHRIFT

(11) **EP 2 271 633 B1**
(45) Veröffentlichungstag und Bekanntmachung des Hinweises auf die Patenterteilung: **27.08.2014**
(21) Anmeldenummer: 09720354.1
(22) Anmeldetag: 12.03.2009
(51) Int. Cl.: C07D 301/12

(54) **VERFAHREN ZUR HERSTELLUNG VON CHIRALEN , -EPOXYKETONEN**
METHOD FOR PRODUCING CHIRAL , -EPOXY KETONES
PROCÉDÉ DE FABRICATION D' , -ÉPOXYCÉTONES CHIRALES

(30) Priorität: 12.03.2008 DE 102008013962
(43) Veröffentlichungstag der Anmeldung: 12.01.2011
(73) Patentinhaber: Studiengesellschaft Kohle MbH, 45470 Mülheim an der Ruhr (DE)
(72) Erfinder: LIST, Benjamin, 45470 Mülheim an der Ruhr (DE); REISINGER, Corinna, 8070 Zürich (CH); WANG WANG, Xing, 45470 Mülheim an der Ruhr (DE)
(86) Internationale Anmeldenummer: PCT/DE2009/000315
(87) Internationale Veröffentlichungsnummer: WO 2009/112014

(56) Entgegenhaltungen:
- WO-A1-2005/077908
- WANG XINGWANG ET AL: "Catalytic asymmetric epoxidation of cyclic enones." JOURNAL OF THE AMERICAN CHEMICAL SOCIETY 14 MAY 2008, Bd. 130, Nr. 19, 14. Mai 2008 (2008-05-14) , Seiten 6070-6071, XP002534893 ISSN: 1520-5126
- LATTANZI ALESSANDRA: "Advances in asymmetric epoxidation of alpha,beta-unsaturated carbonyl compounds: The organocatalytic approach" CURRENT ORGANIC SYNTHESIS, Bd. 5, Nr. 2, Mai 2008 (2008-05), Seiten 117-133, XP009119114 ISSN: 1570-1794
- PORTER M J ET AL: "Asymmetric epoxidation of electron-deficient olefins" CHEMICAL COMMUNICATIONS 20000721 GB, Nr. 14, 21. Juli 2000 (2000-07-21), Seiten 1215-1225, XP002534894 ISSN: 1359-7345
- GENSKI T ET AL: "Epoxidation of electron deficient alkenes using tert-butyl hydroperoxide and 1,5,7-triazabicyclo[4.4,0]dec-5-ene and its Derivatives" SYNLETT 1999 DE, Nr. 6, 1999, Seiten 795-797, XP002534895 ISSN: 0936-5214
- MCMANUS J C ET AL: "Enantiopure guanidine bases for enantioselective enone epoxidations: 1, Acyclic guanidines" SYNLETT 2003 DE, Nr. 3, 2003, Seiten 365-368, XP002534896 ISSN: 0936-5214
- MCMANUS J C ET AL: "Enantiopure guanidine bases for enantioselective enone epoxidations: 2, cyclic guanidines" SYNLETT 2003 DE, Nr. 3, 2003, Seiten 369-371, XP002534897 ISSN: 0936-5214
- LATTANZI ALESSANDRA: "Enantioselective epoxidation of alpha,beta-enones promoted by alpha,alpha-diphenyl-L-prolinol as bifunctional organocatalyst." ORGANIC LETTERS 23 JUN 2005, Bd. 7, Nr. 13, 23. Juni 2005 (2005-06-23), Seiten 2579-2582, XP002534898 ISSN: 1523-7060
- LATTANZI ALESSANDRA: "Bis/3,5-dimethylphenyl)-(S)-pyrrolidin-2- ylmethanol: an Improved Organocatalyst for the Asymmetric Epoxidation of alpha,beta-Enones" ADVANCED SYNTHESIS & CATALYSIS., Bd. 348, 2006, Seiten 339-346, XP002534899 WILEY VCH VERLAG, WEINHEIM. ISSN: 1615-4150
- LI YAWEN ET AL: "4-Substituted-alpha,alpha-diaryl-prolinol s improve the enantioselective catalytic epoxidation of alpha,beta-enones." THE JOURNAL OF ORGANIC CHEMISTRY 5 JAN 2007, Bd. 72, Nr. 1, 5. Januar 2007 (2007-01-05) , Seiten 288-291, XP002534904 ISSN: 0022-3263
- WANG XINGWANG ET AL: "Asymmetric counteranion-directed catalysis for the epoxidation of enals." ANGEWANDTE CHEMIE (INTERNATIONAL ED. IN ENGLISH) 2008, Bd. 47, Nr. 6, 27. Dezember 2007 (2007-12-27), Seiten 1119-1122, XP002534900 ISSN: 1521-3773

## Beschreibung

Die vorliegende Erfindung betrifft ein Verfahren zur Herstellung von chiralen α,β-Epoxyketonen.

Funktionalisierte Epoxide sind sehr wertvolle Intermediate in der Synthese industriell relevanter Verbindungen.

Mögliche Zugänge zu enantiomerenreinen α,β-Epoxyketonen stellen asymmetrische Epoxidierungen der entsprechenden α,β-ungesättigten Ketone dar.

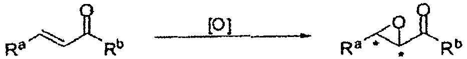

Eine Reihe von Beispielen für diesen Reaktionstyp sind in der Literatur beschrieben worden.

Beispielhaft genannt sind:
WO 2005/077908 Al (TAKASAGO PERFUMERY CO LTD [JP];
TERADA MASAHIRO [JP]: UBE HITOSHI [JP]) 25. August 2005 (2005-08-25);
PORTER M J ET AL: "Asymmetric epoxidation of eleetrondeficient olefins"
CHEMICAL COMMUNICATIONS 20000721 GB, Nr. 14, 21. Juli 2000 (2000-07-21), Seiten 1215-1225;
GENSKI T ET AL: "Epoxidation of electron defieient alkenes using tert-butyl hydroperoxide and 1,5,7-triazabieyelo[4.4,0]dec-5-ene and its Derivatives" SYNLETT 1999 DE, Nr. 6, 1999, Seiten 795-797;
MCMANUS J C ET AL: "Enantiopure guanidine bases for enantioselective enone epoxidations: 1, Acyelic guanidines" SYNLETT 2003 DE, Nr. 3, 2003 , Seiten 365-368;
MCMANUS J C ET AL: "Enantiopure guanidine bases for enantioselective enone epoxidations: 2, cyclic guanidines", SYNLETT 2003 DE, Nr. 3, 2003 , Seiten 369-371;
LATTANZI ALESSANDRA: "Enantioselective epoxidation of alpha,beta-enones promoted by alpha,alpha-diphEmyl-L-prolinol as bifunctional organocatalyst." ORGANIC LETTERS 23.JUN 2005, Bd. 7, Nr. 13, 23. Juni 2005 (2005-06-23), Seiten 2579-2582;
LATTANZI ALESSANDRA: "Bis/3,5-dimethylphenyl)-(S)-pyrrolidin-2-ylmethanol: an Improved Organocatalyst for the Asymmetric Epoxidation of alpha,beta-Enones" ADVANCED SYNTHESIS & CATALYSIS., Bd. 348, 2006, Seiten 339-346, WILEY VCH VERLAG, WEINHEIM;
LI YAWEN ET AL: "4-Substituted-alpha,alpha-diaryl-prolinols improve the enantioselective catalytic epoxidation of alpha,beta enones." THE JOURNAL OF ORGANIC CHEMISTRY 5 JAN 2007, Bd. 72, Nr. 1,5. Januar 2007 (2007-01-05), Seiten 288-291;
WANG XINGWANG ET AL: "Asymmetric counteranion-directed catalysis for the epoxidation of enals." ANGEWANDTE CHEMIE (INTERNATIONAL ED. IN ENGLISH) 2008, Bd. 47, Nr. 6, 27.Dezember 2007(2007-12-27) ; Seiten 1119-1122.

Darunter finden sich zahlreiche Beispiele für die enantioselektive Epoxidierung von Chalcon und Chalconderivaten. (Chem. Commun.) Hoch enantioselektive Epoxidierungen von cyclischen α,β-ungesättigten Ketonen sind jedoch unbekannt. Sowohl mit Hilfe stöchiometrisch eingesetzter chiraler Reagenzien, als auch unter Einsatz chiraler Katalysatoren konnten nur unbefriedigende Enantioselektivitäten erreicht werden. Des Weiteren ist keine generelle Methode für die hoch enantioselektive Epoxidierung aliphatischer α,β-ungesättigter Ketone verfügbar.

Der vorliegenden Erfindung lag die Aufgabe zugrunde, ein einfaches Verfahren zur Herstellung enantiomerenangereicherter cyclischer α,β-Epoxyketone zur Verfügung zu stellen.

Gegenstand der vorliegenden Erfindung ist ein Verfahren zur enantioselektiven Epoxidierung von α,β-ungesättigten Ketonen, in welchem eine Verbindung der allgemeinen Formel I, 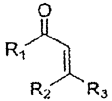 in der
R¹ für einen verzweigten oder unverzweigten, gesättigten oder ungesättigten Kohlenwasserstoffrest mit 1 bis 30 Kohlenstoffatomen, der geeignete Substituenten haben kann und ein oder mehrere Heteroatome in der Kette aufweisen kann,
R² für Wasserstoff, einen verzweigten oder unverzweigten, gesättigten oder ungesättigten Kohlenwasserstoffrest mit 1 bis 30 Kohlenstoffatomen, der geeignete Substituenten haben kann und ein oder mehrere Heteroatome in der Kette aufweisen kann, eine Arylgruppe oder Heteroarylgruppe, die geeignete Substituenten haben kann,
R³ für Wasserstoff, einen verzweigten oder unverzweigten, gesättigten oder ungesättigten Kohlenwasserstoffrest mit 1 bis 30 Kohlenstoffatomen, der geeignete Substituenten haben kann und ein oder mehrere Heteroatome in der Kette aufweisen kann, eine Arylgruppe oder Heteroarylgruppe, die geeignete Substituenten haben kann, stehen,
R¹, R², R³ gleich oder verschieden sein können,
wobei der Rest R¹ mit den Resten R² und R³ einen Ring bilden kann, der 5- bis 20-gliedrig, gesättigt oder ungesättigt, alicyclisch oder heteroalicyclisch sein kann und geeignete Substituenten haben kann,
mit einem Oxidationsmittel unter Bildung von α,β-Epoxyketonen der allgemeinen Formel II, 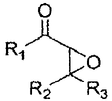 in der R¹, R², R³ wie oben definiert sind,
in Gegenwart eines chiralen Katalysators, ausgewählt aus Aminen der allgemeinen Formel V, VI oder VII und Additionssalzen dieser Amine der allgemeinen Formel V, VI oder VII mit achiralen oder chiralen Säuren, umgesetzt wird,
wobei das chirale Amin ausgewählt ist aus Verbindungen mit den Formeln V, VI und/oder VII 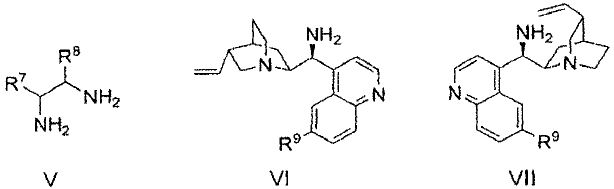 in der
R⁷ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, steht,
R⁸ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, steht,
R⁷, R⁸ gleich oder verschieden sein können, wobei die Rest R⁷ und R⁸ einen Ring bilden können, der 4- bis 20-gliedrig, gesättigt oder ungesättigt, alicyclisch oder heteroalicyclisch sein kann und geeignete Substituenten haben kann, und
R⁹ für H, eine Gruppe -OR¹⁰ steht, in der R¹⁰ für Wasserstoff, eine Kohlenwasserstoffgruppe mit 1 bis 30 Kohlenstoffatomen, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann,
und
wobei die chiralen Säuren ausgewählt sind aus chiralen organischen Phosphorsäuren, Phosphorimiden, Schwefelsäuren, Sulfonsäuren, Sulfonylimiden, Carbonsäuren, und Imiden.

Es wurde gefunden, dass α,β-Epoxyketone der allgemeinen Formel II in guten Ausbeuten und hervorragenden Enantioselektivitäten aus α,β-ungesättigten Ketonen der allgemeinen Formel I durch Epoxidierung mit Wasserstoffperoxid in Gegenwart eines chiralen Katalysators, wie Aminoverbindungen und deren Säureadditonssalze erhalten werden

Zur Durchführung des erfindungsgemäßen Verfahrens werden α,β-ungesättigte Ketone der allgemeinen Formel I in Gegenwart eines chiralen Katalysators mit einem geeigneten Oxidationsmittel umgesetzt. Als Katalyzator werden Amine und deren Säureadditionssalze, erwiesen. Die Additionssalze können per se eingesetzt werden oder sich im Laufe der Reaktion bilden. Die Amine weisen eine Struktur der allgemeinen Formel V, VI oder VII auf.

Der chirale Katalysator ist ausgewählt aus chiralen Aminen der allgemeinen Formel V, VI oder VII, und aus Additionssalzen von chiralen Aminen der allgemeinen Formel V, VI oder VII mit achiralen oder chiralen Säuren.

Im erfindungsgemäßen Verfahren können als achirale Säuren beispielsweise halogenierte Carbonsäuren eingesetzt werden, wie halogenierte Essigsäuren, z. B. Trifluoressigsäure, Trichloressigsäure, Difluoressigsäure und Dichloressigsäure, Benzoesäure, substituierte Benzoesäuren, etc..

Erfindungsgemäß geeignete chirale Säuren sind chirale organische Phosphorsäuren, Phosphorimiden, Schwefelsäuren, Sulfonsäuren, Sulfonylimiden, Carbonsäuren. Imide etc. Vorzugsweise leiten sich die chiralen Säuren von Binaphthol ab. In einer möglichen Ausführungsform ist die chirale Säure ausgewählt aus organischen chiralen Phosphorsäuren mit der allgemeinen Formel IV, 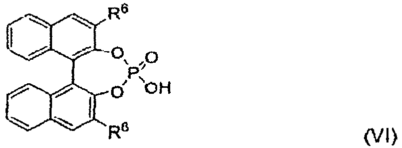 in der
R⁸ für H, eine Kohlenwasserstoffgruppe, wie eine gesättigte oder ungesättigte, verzweigte oder lineare C₁-C₂₀-Alkylgruppe, C₂-C₂₀-Alkenylgruppe, C₂-C₂₀-Alkinylgruppe, A2rylgruppe, die geeignete Substituenten, auch Heteroatomsubstituenten, haben kann, eine Heteroatomenthaitende Kohlenwasserstoffgruppe, die geeignete Substituenten haben kann, steht.

Erfindungsgemäß werden Amine ausgewählt aus den nachfolgenden Verbindungen mit den Formeln V, VI und VII 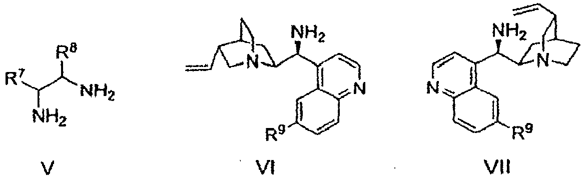 in denen
R⁷ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, und
R⁸ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann,
R⁷, R⁸ gleich oder verschieden sein können,
wobei die Rest R⁷ und R⁸ einen Ring bilden können, der 4- bis 20-gliedrig, gesättigt oder ungesättigt, alicyclisch oder heteroalicyclisch sein kann und geeignete Substituenten haben kann, und
R⁹ für H, eine Gruppe -OR¹⁰,
in der R¹⁰ für Wasserstoff, eine Kohlenwasserstoffgruppe mit 1 bis 30 Kohlenstoffatomen, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, steht, stehen.

Der Katalysator wird üblicherweise in einer Menge von 0,1 bis 200 Mol%, vorzugsweise von 1 bis 30 Mol%, bezogen auf die Ausgangsverbindungen, eingesetzt.

Als Oxidationsmittel kommt insbesondere H₂O₂, das vorzugsweise in wässriger Lösung eingesetzt wird, insbesondere in einer Konzentration über 30 Gew.-%, vorzugsweise zwischen 30 und 50 Gew.-%.

Kohlenwasserstoffgruppe bedeutet im Rahmen der Erfindung eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, oder eine Heteroatom-enthaltende Kohlenwasserstoffgruppe.

Alkyl kann unverzweigt (linear) oder verzweigt sein und hat 1 bis 30, vorzugsweise 1, 2, 3, 4, 5, 6, 7, 8, 9, 10, 11, 12, 13, 14, 15, 16, 17, 18, 19 oder 20 Kohlenstoffatome. Alkyl ist vorzugsweise Methyl, aber auch Ethyl, Propyl, Isopropyl, Butyl, Isobutyl, sec-Botyl oder tert.-Butyl, ebenso Pentyl, 1-, 2- oder 3-Methylpropyl, 1,1-, 1,2- oder 2,2 Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, vorzugsweise aber auch z.B. Trifluormethyl.

Alkyl ist besonders bevorzugt ein Alkyl mit 1, 2, 3, 4, 5 oder 6 Kohlenstoffatomen, vorzugsweise Methyl, Ethyl, Propyl, Isopropyl, Butyl, Isopropyl, sec-Butyl, tert-Butyl, Pentyl, Hexyl, Trifluormethyl, Pentafluroethyl oder 1,1,1-Trifluorethyl.

Cycloalkyl ist vorzugsweise Cyclopropyl, Cyclobutyl, Cyclopentyl, Cyclohexyl oder Cycloheptyl. Alkylen bedeutet vorzugsweise Methylen, Ethylen, Propylen, Butylen, Pentylen oder Hexylen, aber auch verzweigtes Alkylen.

Alkylen ist bevorzugt Vinyl.

Alkinyl ist bevorzugt C≡CH.

Halogen ist F, Cl, G, Br oder I.

Alkoxy ist vorzugsweise Methoxy, Ethoxy, Propoxy oder Butoxy.

C₃-C₈-Heterocycloalkyl mit einem oder mehreren Heteroatomen ausgewählt aus N, O und S ist vorzugsweise 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-piperidinyl, 2-, 3- oder 4-morpholinyl, Tetrahydro-2-, -3- oder -4-pyranyl, 1,4-Dioxanyl, 1,3-Dioxan-2-, -4- oder -5-yl, hexahydro-1-, -3- oder -4-pyridazinyl, Hexahydro-1-, -2-, -4- oder -5-pyrimidinyl, 1-, 2- oder 3-piperazinyl, 1,2,3,4-Tetrahydro-1-, -2-, -3-, -4-, -5-, -6-, -7- oder -8-chinolyl, 1,2,3,4Tetrahydro-1-, -2-, -3-, -4-, -5-, -6-, -7- oder -8-isochinolyl, 2-, 3-, 5-, 6-, 7- oder 8-3,4-Dihydro-2H-benzo-1,4-oxazinyl.

Gegebenenfalls substituiert bedeutet unsubstituiert oder mono-, di-, tri-, tetra- oder pentasubstituiert.

Aryl ist vorzugsweise Phenyl, Naphthyl oder Diphenyl.

Arylalkyl ist vorzugsweise Benzyl.

Heteroaryl mit einem oder mehreren Heteroatomen ausgewählt aus N, O und S ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4-oder 5-Thiazolyl, 3-, 4- oder 5-Isothiazolyl, 2-, 3- oder 4-Pyridyl, 2-, 4-, 5- oder 6-Pyrimidinyl, außerdem bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2-oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo-1,4-oxazinyl, außerdem bevorzugt 1,3-Benzodioxol-5-yl, 1,4-Benzodioxan-6-yl, 2,1,3-Benzothiadiazol-4-oder -5-yl oder 2,1,3-Benzoxadiazol-5-yl.

Beispiele für Substituenten sind C₁-C₄-Alk(en)yl, Aryl, Heteroaryl, Halogen, wie F, Cl, Br, I, NO₂, Amino, usw.

Die Reaktion kann in üblichen polaren oder unpolaren organischen Lösungsmitteln durchgeführt werden.

### Beispiele

### A. Allgemeine Vorschrift:

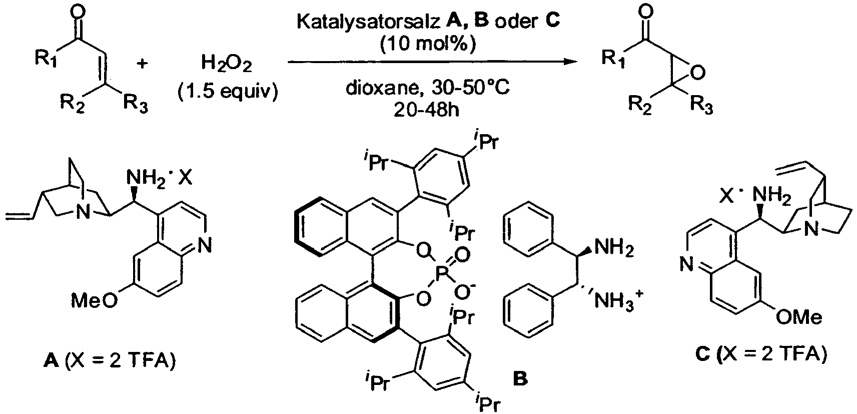

Die Katalysatorsalze A-C wurden in situ in Dioxan (2-4 mL) aus dem Amin (10 mol%) und der jeweiligen Säure (10-20 mol%) dargestellt. Nach 20 min Rühren wurden die α,β-ungesättigten Ketone zugegeben und nach weiteren 20 min wurden 1.5 Äquivalente einer wässrigen Wasserstoffperoxidlösung (50% w/w) zugesetzt. Nach einer Reaktionszeit von 20-72 h bei 30-50 °C wurde das Reaktionsgemisch abgekühlt und mit Wasser versetzt. Anschließend wurde mit Ether extrahiert, dann die vereinigten organischen Phasen mit gesättigter Natriumchloridlösung gewaschen, getrocknet (Na₂SO₄), filtriert und am Rotationsverdampfer eingeengt, wodurch die Rohprodukte erhalten wurden, welche chromatographisch (SiO₂, Ether/Pentan) aufgereingt wurden. Im Fall des acyclischen α,β-ungesättigten Ketons wurde das derart erhaltene Rohprodukt gegebenenfalls 10 Minuten bis 1 Stunde in Ether mit einem Äquivalent 1 N NaOH-Lösung gerührt. Anschließend wurde die Etherphase mit gesättigter Natriumchloridlösung gewaschen, getrocknet (Na₂SO₄), filtriert und am Rotationsverdampfer eingeengt. Anschließend wurde chromatographisch (SiO₂, Ether/Pentan) aufgereingt.
**Mit Katalysator A:** 1.0 mmol Maßstab bezogen auf das α,β-ungesättigte Keton. Das Katalysatorsalz **A** wurde aus 9-amino-9-deoxyepiquinine (8.1 mg, 0.1 mmol, 10 mol%) und TFA (15.3 µL, 0.2 mmol, 20 mol%) dargestellt.
**Mit Katalysator B:** 0.5 mmol Maßstab bezogen auf das α,β-ungesättigte Keton. Das Katalysatorsalz **B** wurde aus (R,R)-DPEN (10.6 mg, 0.05 mmol, 10 mol%) and S-TRIP (37.6 mg, 0.05 mmol, 10 mol%).
**Mit Katalysator C:** 1.0 mmol Maßstab bezogen auf das α,β-ungesättigte Keton. Das Katalysatorsalz **C** wurde aus 9-amino-9-deoxyepiquinidine (8.1 mg, 0.1 mmol, 10 mol%) und TFA (15.3 µL, 0.2 mmol, 20 mol%) dargestellt.

**Tabelle I: Herstellung von cyclischen Epoxiden**

| Beispiel | Epoxid | Katalysator | Ausbeute (%) | *er* |
|---|---|---|---|---|
| 1 | 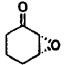 | **B** | 98 | 96:4 |
| 2 | | **A** | 91 | 3:97 |
| 3 | 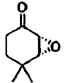 | **B** | 80 | 97:3 |
| 4 | 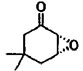 | **B** | 76 | 98:2 |
| 5 | 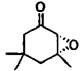 | **B** | 63 | 96:4 |
| 6 | 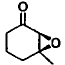 | **A** | 70 | 98:2 |
| 7 | 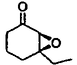 | **A** | 73 | 98.5:1.5 |
| 8 | 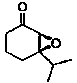 | **A** | 79 | 99:1 |
| 9 | 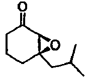 | **A** | 73 | 98:2 |
| 10 | 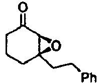 | **A** | 84 | 98.5:1.5 |
| 11 | 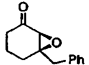 | **A** | 78 | 99:1 |
| 12 | 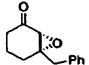 | **C** | 77 | 98.5:1.5 |
| 13 | 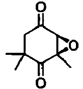 | **A** | 49 | 96:4 |
| 14 | 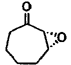 | **B** | 82 | 99:1 |
| 15 | 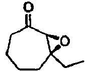 | **A** | 82 | >99.5:0.5 |
| 16 | 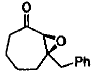 | **A** | 85 | >99.5:0.5 |
| 17 | 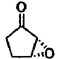 | **B** | 29 | 89:11 |

**Tabelle 2: Herstellung von alicyclischen Epoxiden**

| | | | | | | |
|---|---|---|---|---|---|---|
| 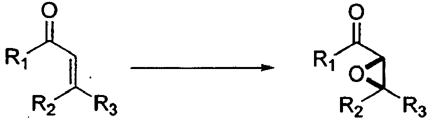 | | | | | | |

| Beispiel | R¹ | R² | R³ | Katalysator | Ausbeute (%) | *er* |
|---|---|---|---|---|---|---|
| 18 | nC₆H₁₃ | H | Me | **A** | 72 | 98.5:1.5 |
| 19 | 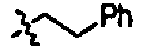 | H | Me | **A** | 85 | 98.5:1.5 |
| 20 | 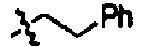 | Me | H | **A** | 82 | 97.5:2.5 |
| 21^{a} | 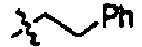 | H | Me | **C** | 90 | 95:5 |
| 22 | 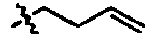 | H | Me | **A** | 76 | 98.5:1.5 |
| 23 | *i*Bu | H | Me | **A** | 77 | 98.5:1.5 |
| 24 | Cy | H | Me | **A** | 83 | 98.5:1.5 |
| 25 | 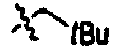 | H | Me | **A** | 81 | >99.5:0.5 |
| 26 | Me | H | Et | **A** | 55 | 98.5:1.5 |
| 27 | *n*C₉H₁₉ | H | Et | **A** | 82 | 99:1 |
| 28 | *n*C₅H₁₁ | H | *n*C₅H₁ 1 | **A** | 76 | 99:1 |
| 29 | *n*C₅H₁₁ | H | *i*BU | **A** | 81 | 98.5:1.5 |

| | | | | | | |
|---|---|---|---|---|---|---|
| ^{a}Mit Katalysator C wird das entgegengesetzte Enantiomer erhalten. | | | | | | |

## Patentansprüche

1. Verfahren zur enantioselektiven Epoxidierung von α,β-ungesättigten Ketonen, in welchem eine Verbindung der allgemeinen Formel I, 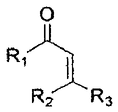 in der
R¹ für einen verzweigten oder unverzweigten, gesättigten oder ungesättigten Kohlenwasserstoffrest mit 1 bis 30 Kohlenstoffatomen, der geeignete Substituenten haben kann und ein oder mehrere Heteroatome in der Kette aufweisen kann,
R² für Wasserstoff, einen verzweigten oder unverzweigten, gesättigten oder ungesättigten Kohlenwasserstoffrest mit 1 bis 30 Kohlenstoffatomen, der geeignete Substituenten haben kann und ein oder mehrere Heteroatome in der Kette aufweisen kann, eine Arylgruppe oder Heteroarylgruppe, die geeignete Substituenten haben kann,
R³ für Wasserstoff, einen verzweigten oder unverzweigten, gesättigten oder ungesättigten Kohlenwasserstoffrest mit 1 bis 30 Kohlenstoffatomen, der geeignete Substituenten haben kann und ein oder mehrere Heteroatome in der Kette aufweisen kann, eine Arylgruppe oder Heteroarylgruppe, die geeignete Substituenten haben kann, stehen,
R¹, R², R³ gleich oder verschieden sein können,
wobei der Rest R¹ mit den Resten R² und R³ einen Ring bilden kann, der 5- bis 20-gliedrig, gesättigt oder ungesättigt, alicyclisch oder heteroalicyclisch sein kann und geeignete Substituenten haben kann,
mit einem Oxidationsmittel unter Bildung von α,β-Epoxyketonen der allgemeinen Formel II, 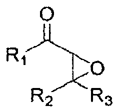 in der R¹, R², R³ wie oben definiert sind,
in Gegenwart eines chiralen Katalysators, ausgewählt aus Aminen der allgemeinen Formel V, VI oder VII und Additionssalzen dieser Amine der allgemeinen Formel V, VI oder VII mit achiralen oder chiralen Säuren, umgesetzt wird,
wobei das chirale Amin ausgewählt ist aus Verbindungen mit den Formeln V, VI und/oder VII 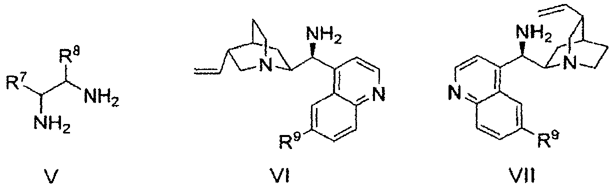 in der
R⁷ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, steht,
R⁸ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, steht,
R⁷, R⁸ gleich oder verschieden sein können, wobei die Rest R⁷ und R⁸ einen Ring bilden können, der 4- bis 20-gliedrig, gesättigt oder ungesättigt, alicyclisch oder heteroalicyclisch sein kann und geeignete Substituenten haben kann, und
R⁹ für H, eine Gruppe -OR¹⁰ steht, in der R¹⁰ für Wasserstoff, eine Kohlenwasserstoffgruppe mit 1 bis 30 Kohlenstoffatomen, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann,
und
wobei die chiralen Säuren ausgewählt sind aus chiralen organischen Phosphorsäuren, Phosphorimiden, Schwefelsäuren, Sulfonsäuren, Sulfonylimiden, Carbonsäuren, und Imiden.

2. Verfahren nach Anspruch 1, **dadurch gekennzeichnet, dass** das Oxidationsmittel aus Wasserstoffperoxid und -formulierungen, Alkylperoxiden und Alkylperoxidformulierungen, Natriumhypochlorit, Persäuren, Iodosoverbindungen, Boraten ausgewählt ist.

3. Verfahren nach Anspruch 2, **dadurch gekennzeichnet, dass** als Oxidationsmittel eine wässrige Wasserstoffperoxidlösung eingesetzt wird.

4. Verfahren nach einem der Ansprüche 1 bis 3, **dadurch gekennzeichnet, dass** sich die chirale Säure von Binaphthol ableitet.

5. Verfahren nach Anspruch 4, **dadurch gekennzeichnet, dass** die chirale Säure ausgewählt ist aus organischen chiralen Phosphorsäuren mit der allgemeinen Formel IV, 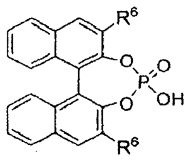 in der
R⁶ für H, eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, steht.

## Claims

1. Process for the enantioselective epoxidation of α,β -unsaturated ketones, in which a compound of the general formula 1, 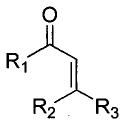 in which
R¹ is a branched or unbranched, saturated or unsaturated hydrocarbon radical having 1 to 30 carbon atoms, which may have suitable substituents and may have one or more heteroatoms in the chain,
R² is hydrogen, a branched or unbranched, saturated or unsaturated hydrocarbon radical having 1 to 30 carbon atoms, which may have suitable substituents and may have one or more heteroatoms in the chain, an aryl group or heteroaryl group, which may have suitable substituents,
R³ is hydrogen, a branched or unbranched, saturated or unsaturated hydrocarbon radical having 1 to 30 carbon atoms, which may have suitable substituents and may have one or more heteroatoms in the chain, an aryl group or heteroaryl group, which may have suitable substituents,
R¹, R², and R³ may be identical or different,
and the radical R¹ may, with the radicals R² and R³, form a ring, which may have 5 to 20 members, be saturated or unsaturated, alicyclic or heteroalicyclic, and may have suitable substituents,
is reacted with an oxidizing agent to form α,β-epoxy ketones of the general formula II, 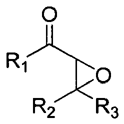 in which R¹, R², R³ are as defined above.
wherein the reaction is carried out in the presence of a chiral catalyst, being selected from amines having the formulae V, VI and/or VII and addition salts of the amines of the general formula V, VI or VII with achiral or chiral acids, 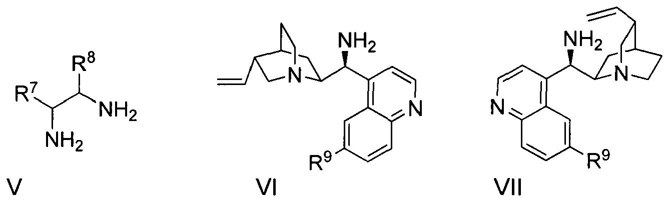 in which
R⁷ is a hydrocarbon group, such as a saturated or unsaturated, branched or linear alkyl group, alkenyl group, alkynyl group or aryl group, which may have suitable substituents, including heteroatom substituents, or a heteroatom-containing hydrocarbon group, which may have suitable substituents, and
R⁸ is a hydrocarbon group, such as a saturated or unsaturated, branched or linear alkyl group, alkenyl group, alkynyl group or aryl group, which may have suitable substituents, including heteroatom substituents, or a heteroatom-containing hydrocarbon group, which may have suitable substituents,
R⁷ and R⁸ may be identical or different, wherein the radicals R⁷ and R⁸ may form a ring, which may have 4 to 20 members, be saturated or unsaturated, alicyclic or heteroalicyclic, and may have suitable substituents, and
R⁹ is H, or a group -OR¹⁰ in which R¹⁰ is hydrogen, a hydrocarbon group having 1 to 30 carbon atoms, such as a saturated or unsaturated, branched or linear alkyl group, alkenyl group, alkynyl group or aryl group, which may have suitable substituents, including heteroatom substituents, or a heteroatom-containing hydrocarbon group, which may have suitable substituents, and
wherein the chiral acids are selected from chiral organic phosphoric acids, phosphorimides, sulfuric acids, sulfonic acids, sulfonylimides, carboxylic acids and imides.

2. The process as claimed in claim 1, **characterized in that** the oxidizing agent is selected from hydrogen peroxide and its formulations, alkyl peroxides and alkyl peroxide formulations, sodium hypochlorite, peracids, iodoso compounds and borates.

3. The process as claimed in claim 2, **characterized in that** an aqueous hydrogen peroxide solution is used as oxidizing agent.

4. The process as claimed in any of claims 1 to 3, **characterized in that** the chiral acid is derived from binaphthol.

5. The process as claimed in claim 4, **characterized in that** the chiral acid is selected from organic chiral phosphoric acids having the general formula IV 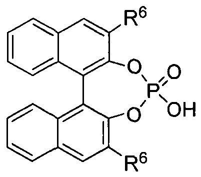 in which R⁶ is H, a hydrocarbon group, saturated or unsaturated, branched or linear alkyl group, alkenyl group, alkynyl group or aryl group which may have suitable substituents, including heteroatom substituents, or a heteroatom-containing hydrocarbon group, which may have suitable substituents.

## Revendications

1. Procédé d'époxydation énantiosélective de cétones α,β-insaturées, selon lequel un composé de formule générale I 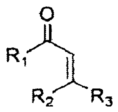 dans laquelle
R¹ représente un radical hydrocarboné ramifié ou non ramifié, saturé ou insaturé, de 1 à 30 atomes de carbone, qui peut comprendre des substituants appropriés et qui peut comprendre un ou plusieurs hétéroatomes dans la chaîne,
R² représente l'hydrogène, un radical hydrocarboné ramifié ou non ramifié, saturé ou insaturé, de 1 à 30 atomes de carbone, qui peut comprendre des substituants appropriés et qui peut comprendre un ou plusieurs hétéroatomes dans la chaîne, un groupe aryle ou un groupe hétéroaryle, qui peut comprendre des substituants appropriés,
R³ représente l'hydrogène, un radical hydrocarboné ramifié ou non ramifié, saturé ou insaturé, de 1 à 30 atomes de carbone, qui peut comprendre des substituants appropriés et qui peut comprendre un ou plusieurs hétéroatomes dans la chaîne, un groupe aryle ou un groupe hétéroaryle, qui peut comprendre des substituants appropriés,
R¹, R², R³ peuvent être identiques ou différents,
le radical R¹ pouvant former un cycle avec les radicaux R² et R³, qui peut être de 5 à 20 éléments, être saturé ou insaturé, alicyclique ou hétéroalicyclique, et qui peut comprendre des substituants appropriés,
est mis en réaction avec un oxydant pour former des α,β-époxycétones de formule générale II 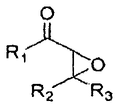 dans laquelle R¹, R², R³ sont tels que définis précédemment,
en présence d'un catalyseur chiral, choisi parmi les amines de formule générale V, VI ou VII et les sels d'addition de ces amines de formule générale V, VI ou VII avec des acides achiraux ou chiraux,
l'amine chirale étant choisie parmi les composés de formule V, VI et/ou VII 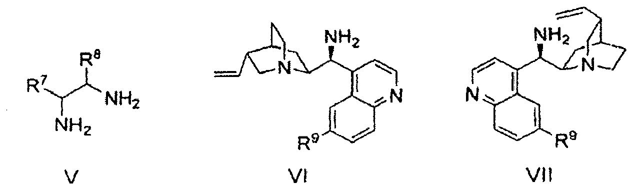 dans lesquelles
R⁷ représente un groupe hydrocarboné, tel qu'un groupe alkyle saturé ou insaturé, linéaire ou ramifié, un groupe alcényle, un groupe alcynyle, un groupe aryle, qui peut comprendre des substituants appropriés, également des substituants hétéroatomes, un groupe hydrocarboné contenant des hétéroatomes, qui peut comprendre des substituants appropriés,
R⁸ représente un groupe hydrocarboné, tel qu'un groupe alkyle saturé ou insaturé, linéaire ou ramifié, un groupe alcényle, un groupe alcynyle, un groupe aryle, qui peut comprendre des substituants appropriés, également des substituants hétéroatomes, un groupe hydrocarboné contenant des hétéroatomes, qui peut comprendre des substituants appropriés,
R⁷, R⁸ peuvent être identiques ou différents, les radicaux R⁷ et R⁸ pouvant former un cycle, qui peut être de 4 à 20 éléments, être saturé ou insaturé, alicyclique ou hétéroalicyclique, et qui peut comprendre des substituants appropriés, et
R⁹ représente H, un groupe -OR¹⁰, dans lequel R¹⁰ représente l'hydrogène, un groupe hydrocarboné de 1 à 30 atomes de carbone, tel qu'un groupe alkyle saturé ou insaturé, linéaire ou ramifié, un groupe alcényle, un groupe alcynyle, un groupe aryle, qui peut comprendre des substituants appropriés, également des substituants hétéroatomes, un groupe hydrocarboné contenant des hétéroatomes, qui peut comprendre des substituants appropriés,
et
les acides chiraux étant choisis parmi les acides phosphoriques organiques chiraux, les phosphorimides, les acides sulfuriques, les acides sulfoniques, les sulfonylimides, les acides carboxyliques et les imides.

2. Procédé selon la revendication 1, **caractérisé en ce que** l'oxydant est choisi parmi le peroxyde d'hydrogène et les formulations de peroxyde d'hydrogène, les peroxydes d'alkyle et les formulations de peroxyde d'alkyle, l'hypochlorite de sodium, les peracides, les composés iodoso, les borates.

3. Procédé selon la revendication 2, **caractérisé en ce qu'**une solution aqueuse de peroxyde d'hydrogène est utilisée en tant qu'oxydant.

4. Procédé selon l'une quelconque des revendications 1 à 3, **caractérisé en ce que** l'acide chiral est dérivé de binaphtol.

5. Procédé selon la revendication 4, **caractérisé en ce que** l'acide chiral est choisi parmi les acides phosphoriques chiraux organiques de formule générale IV 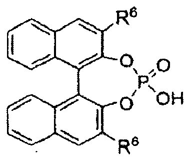 dans laquelle
R⁶ représente H, un groupe hydrocarboné tel qu'un groupe alkyle saturé ou insaturé, linéaire ou ramifié, un groupe alcényle, un groupe alcynyle, un groupe aryle, qui peut comprendre des substituants appropriés, également des substituants hétéroatomes, un groupe hydrocarboné contenant des hétéroatomes, qui peut comprendre des substituants appropriés.
